# EUROPEAN PATENT APPLICATION

(11) **EP 3 093 660 A1**
(43) Date of publication of application: **16.11.2016**
(21) Application number: 15190277.2
(22) Date of filing: 16.10.2015
(51) Int. Cl.: G01N 33/00, G01N 15/14, G01N 15/06

(54) **HYBRID ENVIRONMENT SENSOR**

(30) Priority: 14.05.2015 KR 20150067386
(71) Applicant: Center for Integrated Smart Sensors Foundation, Daejeon (KR)
(72) Inventor: CHO, Hyun Tae, Daejeon (KR)
(74) Representative: Taor, Simon Edward William

(57) **Abstract**

The present description relates to a hybrid environment sensor comprising a particulate matter measuring sensor (20) and a gas sensor unit (10).

## Description

The present description relates to a hybrid environment sensor to measure a concentration of a particulate matter and to detect a gas element. Further the present description relates to a hybrid environment sensor that can reduces the size of the entire environment sensor and the total electric consumption through simultaneously arranging a particulate matter measuring unit and a gas element measurement unit in the entire housing unit.

As living standard gradually improved according to economic development, interest regarding air quality increased and users now pursue a more pleasant and clean air.

Accordingly, the quality of houses has been improving through continuously applying various functions in pursuit of high quality of life. Particularly, various kinds of air cleaner is released and in sale due to health issues caused by particulate matters and foreign substances included in the air emitted from environment polluting machines or automobiles. However, air quality is not improved enough with an air cleaner.

Accordingly, for the health and convenience of the user, technical development that quickly improves air quality based on simple and accurate evaluation of air quality through real-time observation is required.

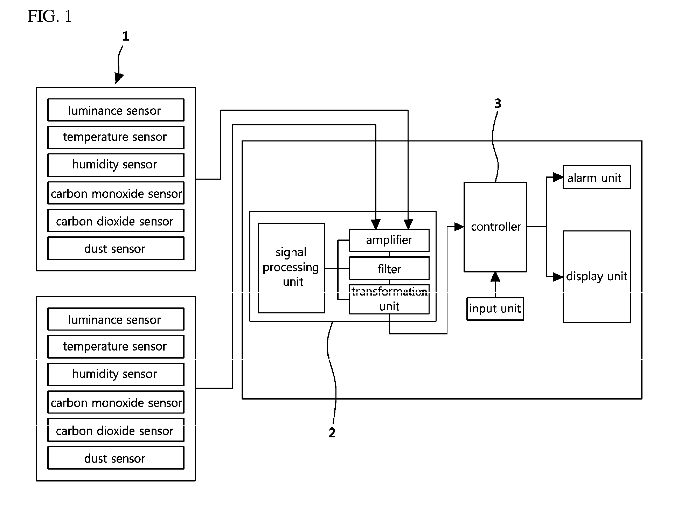

Korea Patent Registration No. 10-1160986 is suggested as an example of a related art. Further, FIG. 1 is the figure accompanying the abstract of Korea Patent Registration 10-1160986.

Referring to FIG. 1, an indoor air pollution measuring device disclosed in Korea Patent Registration No. 10-1160986 includes a detecting unit 1 configured to detect indoor air conditions; a signal processing unit 2 configured to convert the received measured signal that is detected in the detecting unit 1 to a digital data after amplifying and filtering; a control unit 3 receives measured data through the signal processing unit 2 and displays data measured by previously stored data display algorithm and analysis algorithm regarding the measured data and controls alarm and display based on the comparative analysis. Herein, the detecting unit 1 includes a temperature sensor, a humidity sensor, a carbon monoxide sensor, a carbon dioxide sensor, and a dust sensor and each sensor respectively senses the temperature, humidity, an amount of carbon monoxide, carbon dioxide and dust.

Accordingly, the level of indoor air pollution can be measured accurately and displays the analyzed indoor air objectively thereby, arouses the importance of indoor environment and the user becomes more familiar with the device.

However, there is a problem that the size of the entire measuring device increases proportionate to the number of added sensors when a plurality of sensors is formed independently like a related art. Further, electric power amount required to drive respective sensors increases.

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter. Examples overcome the above disadvantages and other disadvantages not described above. Also, the examples are not required to overcome the disadvantages described above, and an example potentially does not overcome any of the problems described above.

In an effort to resolve the afore-mentioned problem, the present description discloses a hybrid environment sensor that reduced the size of the entire sensor system compared to a related art.
Further, the present description relates to a hybrid environment sensor that can reduce electric energy consumption through reducing the number of required devices as a whole.

Further, the present description relates to a hybrid environment sensor that may reduce the manufacture cost by reducing the number of devices required in the entire measuring device through organically arranging the environment sensors.

A hybrid environment sensor according to an embodiment of the present description includes a gas sensor unit configured to detect at least one gas element included in an inflowing air; and a particulate matter measuring sensor unit configured to measure a concentration of the particulate matter in the air moved by the gas sensor unit.

For example, the gas sensor unit may include a gas sensor module configured to detect at least one gas element that is selected from a group including a Volatile Organic Compound (VOC), Nitrogen Oxide (NOₓ), carbon monoxide (CO), carbon dioxide (CO₂), sulfur dioxide (SOₓ), ozone (O₃).

Further, the gas sensor unit may include a micro-heater.

The hybrid environment sensor according to the present description may further include a housing unit with an inlet and an outlet wherein the air flows into the inlet and emits from the outlet and, the gas sensor unit and particulate matter measurement sensor unit may be arranged in the housing unit

The gas sensor unit detects at least one gas element that is included in the air flowing through the inlet of the housing unit and,

The particulate matter measuring sensor unit can be arranged in the region between the gas sensor unit and the outlet.

According to an embodiment of the present description, the gas sensor unit may be arranged adhering to a bottom or side surface of the housing unit.

The hybrid environment sensor according to an embodiment of the present description may further include a pump configured for the air to be pumped into the inlet.

The particulate matter measuring sensor unit may have various technical structures.

For example, the particulate matter measuring sensor unit may include a light source unit, a light detecting unit configured to generate an electric signal corresponding to a detected light scattered by the particulate matter included in the air, and the light is emitted from the light source unit, and a calculating unit configured to calculate a concentration of the particulate matter by using the electric signal that is detected through the light detecting unit.

Herein, the light source unit can include at least one that is selected from a white light diode, an infrared LED diode, and a laser diode.

The particulate matter measurement sensor unit may include a filter configured to filter the particulate matter in the air; and the calculating unit configured to calculate the concentration of the particulate matter using weight information of the particulate matter that is filtered by the filter.

According to the hybrid environment sensor the gas sensor unit generates heat energy during operation process and the air can be moved by the factor including the heat energy.

A hybrid environment sensor according to an embodiment of the present description may detect a concentration of a particulate matter and gas elements in the air with the portable device by combining with a portable device including a light source unit and a camera module.

The hybrid environment sensor according to an embodiment of the present description may include a housing unit combined with the portable device and includes an inlet that air flows inside and an outlet configured to emit the air that passed the inside, a gas sensor unit that is arranged in the housing unit and detects at least one gas element that included in the air flowing in through the inlet, and provides the information to the portable device, a light induction unit, a light induction unit that induces the light that is irradiated from the light source unit of the portable device to be irradiated on the air moved by the gas sensor unit, and a light detecting induction unit configured to induce a camera module of the portable device to detect the light that is scattered by the particulate matter included in the air through irradiated by the light induction unit.

Further, the light detecting induction unit comprising a lens module configured to converge the light that is scattered by fine-dust included in the air.

The hybrid environment sensor according to an embodiment of the present description includes a housing unit including an inlet that air flows inside and an outlet configured to emit air that passed the inside, a gas sensor unit that is arranged in the housing unit and detects at least one gas element that included in the air flowing in through the inlet, a light source unit that is arranged in the housing unit and irradiates light on the air flowing by the heat that is generated by the gas sensor; a light detecting unit configured to generate an electric signal corresponding to a detected light scattered by the particulate matter included in the air, and the light is irradiated from the light source unit; and a calculating unit configured to calculate particulate matter concentration by using electric signal that is detected through the light detecting unit.

According to an aspect of the invention, there is provided a hybrid environment sensor as set out in claim 1. Preferred features are set out in claims 2 to 13.

A hybrid environment sensor according to an embodiment of the present description can simultaneously measure a gas element, a concentration of surrounding air and a concentration of a particulate matter included in the air through arranging a gas sensor unit configured to detect at least one gas element and a fine dust measuring sensor unit together in a sealed housing structure.

Further, a hybrid environment sensor according to an embodiment of the present description can control the direction of the air during operation process of the measuring device using heat generation.

Accordingly, manufacture cost can be reduced since special technical features to control the air flow in the measuring device are not required.
FIG. 1 illustrates a related art according to an embodiment of the present description.
FIG. 2 illustrates a schematic view of a hybrid environment sensor according to an embodiment of the present description.
FIG. 3 illustrates a diagram of a hybrid environment sensor according to an embodiment of the present description.
FIG. 4 illustrates a diagram of a hybrid environment sensor according to an embodiment of the present description.

The following detailed description is provided to assist the reader in gaining a comprehensive understanding of the methods, apparatuses, and/or systems described herein. However, various changes, modifications, and equivalents of the methods, apparatuses, and/or systems described herein will be apparent to one of ordinary skill in the art. The sequences of operations described herein are merely examples, and are not limited to those set forth herein, but may be changed as will be apparent to one of ordinary skill in the art, with the exception of operations necessarily occurring in a certain order. Also, descriptions of functions and constructions that are well known to one of ordinary skill in the art may be omitted for increased clarity and conciseness.

The features described herein may be embodied in different forms, and are not to be construed as being limited to the examples described herein. Rather, the examples described herein have been provided so that this disclosure will be thorough and complete, and will convey the full scope of the disclosure to one of ordinary skill in the art.

In the following description, the same drawing reference numerals are used for the same elements, even in different drawings. The matters defined in the description, such as detailed constructions of terms and elements, are provided to assist in a comprehensive understanding of the present examples. Accordingly, it is apparent that it is possible for the examples to be carried out without those specifically defined matters. Also, well-known functions or constructions are not described in detail to avoid obscuring the examples with unnecessary detail.

While the expressions such as "first" or "second" are potentially used to refer to various elements, the elements are not intended to be limited by the expressions. Such expressions are used only for the purpose of distinguishing one element from the other when referring to such elements.

The expressions presented are used herein only for the purpose of explaining specific examples and are not intended to place limits on the present examples. An expression in singular form also encompasses plural meaning, unless otherwise specified. Throughout the description, the expression "comprise" or "have" is used only to designate the existence of a characteristic, number, step, operation, element, component or a combination thereof which is described herein, but not to preclude possibility of existence of one or more of the other characteristics, numbers, steps, operations, elements, components or combinations of these or other appropriate additions.

Spatial words, such as below, beneath, lower, above and upper are used to conveniently recite a correlation between one element or features with respect to other elements or features, as illustrated in the drawings. When spatial terminology is used with a direction as illustrated in the drawing, if the illustrated element is upside down, the element that was recited as below and beneath is also potentially considered to be above or upper of another element. Thus, examples presented below include all such instances related to the directions of below and above. An element is also potentially aligned in another direction, and thereby spatial words are interpreted according to the alignment. Certain examples are now described in greater detail with reference to the accompanying drawings.

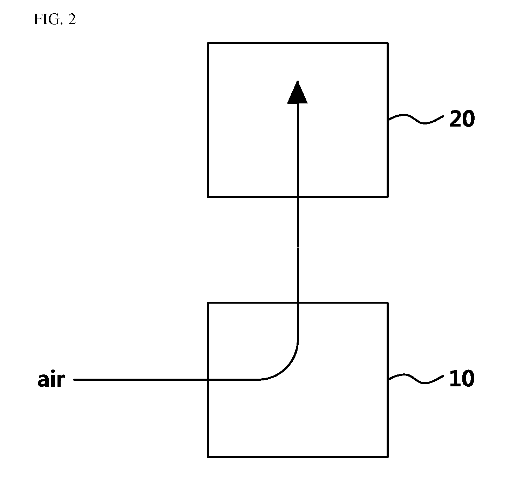

FIG. 2 illustrates a schematic view of a hybrid environment sensor according to an embodiment of the present description.

Referring to FIG. 2, a hybrid environment sensor according to an embodiment of the present description comprises a gas sensor unit 10 and a particulate matter measuring sensor unit 20.

First, the gas sensor unit 10 according to an embodiment of the present description may apply any technical features that may detect gas elements included in the air. For example, the gas sensor unit 10 may include a gas sensor module configured to detect at least one gas element that is selected from a group including a Volatile Organic Compound (VOC), Nitrogen Oxide (NOₓ), carbon monoxide (CO), carbon dioxide (CO₂), sulfur dioxide (SOₓ), and ozone (O₃). Herein, the gas sensor module may include one gas sensor or a plurality of gas sensor according to a type of gas element to measure.

The gas sensor module herein should be heated with over a predetermined temperature to detect a respectively specialized gas element. Generally, a gas sensor module can measure related gas based on changes such as resistance, electrical conductivity and etc. of the gas sensor module surface when a gas particle is absorbed on the surface. Next, re-measuring is possible when the gas molecules absorbed in the sensor surface is removed to detect other gas particle. Therefore, operation of separating the absorbed gas molecules by heating with over predetermined temperature is required.

The gas sensor unit 10 according to an embodiment of the present description may include an additional heating module, more preferably a micro heater to separate gas molecules that is absorbed in the sensor surface to measure a gas element. Through this, a size of the gas sensor unit 10 can be minimized and the size of the hybrid environment including the gas sensor unit 10 can also be minimized.

Likewise, the air is moved by a gas element detecting method of a gas sensor unit 10. Accordingly, the air is moved to pass a particulate matter measuring sensor unit 20. Herein, there are various causes to the move of the air. For example, the air may be moved due to the combination of an additionally equipped pump module and heat energy generated from the gas sensor unit 10. There may be various causes to the move of the air applied in the present description.

The particulate measuring sensor unit 20 can measure a concentration of a particulate matter of air moved by the gas sensor unit 10 configured to measure of. Herein, the particulate matter measuring sensor unit 20 can measure concentration of the particulate matter in the air with methods such as a gravimetric method, a beta-ray absorption method, a light scattering method, a light transmission method and etc.

A gravimetric method filters a predetermined amount of air using a filter and measures a concentration of particulate matter in the air using the weight difference of before and after filtering. Further, a beta-ray absorption method captures particulate matter that float in the air on the filter and transmits beta-ray on the particulate matter thereby, continuously measures the weight concentration of particle material.

A light scattering method measures an amount of light scattering using principle of light scattering when light is irradiated on a particle material and measures a concentration of a particulate matter based on the value.

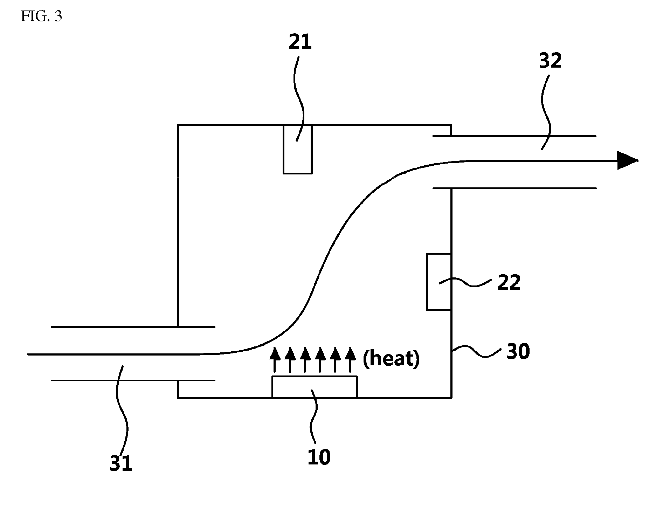

Hereinafter, FIG. 3 illustrates particulate matter measuring sensor unit 20 applied with a light scattering method. However, any skilled person in the related art would understand that other measuring method and necessary technical elements can be applied to the particulate matter measuring sensor unit 20.

According to an embodiment of the present description, when the particulate matter measuring sensor unit 20 measures a concentration of a particulate matter using the gravimetric method, the particulate matter measuring sensor unit 20 may comprise a filter configured to filter particulate matters in the air and an calculating unit configured to calculate the concentration of particulate matters using the weight information of the particulate matters filtered by the above filter.

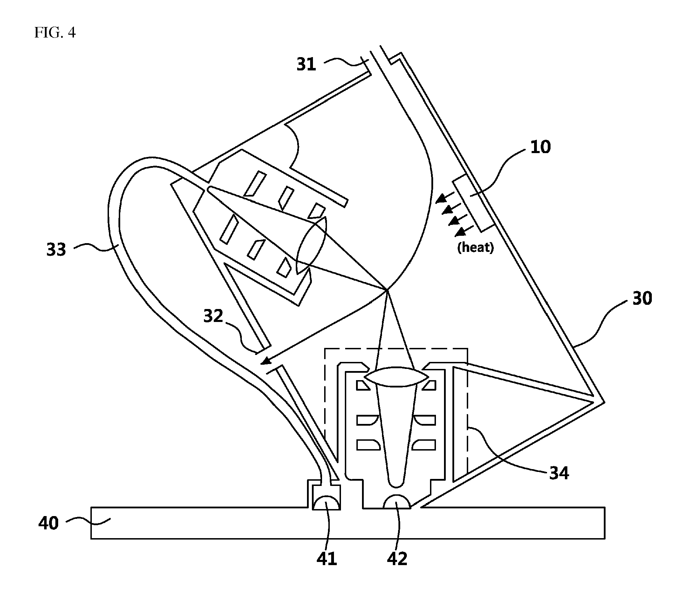

Hereinafter, FIG. 3 and FIG. 4 illustrate a hybrid environment sensor according to an embodiment of the present description.

FIG. 3 illustrates a diagram of a hybrid environment sensor according to an embodiment of the present description.

As illustrated in FIG. 3, the hybrid environment sensor according to the present description my comprise a gas sensor unit 10, a particulate matter measuring sensor unit comprising a light source unit 21 and a light detecting unit 22, and a housing unit 30. Herein, the gas sensor unit 10 and the particulate measuring sensor unit is equipped in the housing unit 30.

The housing unit 30 includes an inlet 31 configured for the air to flow in and an outlet 32 configured for the air that pass the inner housing unit to flow out.

The gas sensor unit 10 is arranged in such housing unit 30 and detects at least one gas element included in the air that flow in through the inlet 31. Herein, the detected gas element can be selectively applied according to types of the gas sensor unit 10 and at least one sensor module. For example, the gas sensor unit 10 may be formed with a sensor module including all sulfur dioxide (Sox) sensor, carbon monoxide (CO) sensor, and ozone (O₃) sensor.

The gas sensor unit 10 is heated with over a predetermined temperature to remove the gas element that is absorbed on the sensor surface during the operation process and the gas sensor unit 10 moves the inflowing air. As illustrated in FIG. 3, when the gas sensor unit 10 is arranged in the bottom surface of the housing unit 30 close to an inlet 31 of the housing unit 30, the inflowing air from the inlet 31 passes the gas sensor unit 10 and moves to the upper direction of the housing unit due to additional causes of air flow including heat energy obtained from the gas sensor unit 10. The moving air flows out through an outlet 32 passing the particulate measuring sensor unit including a light source unit 21 and a light detecting unit 22.

To sum up, a hybrid environment sensor according to an embodiment of the present description includes a gas sensing unit 10 formed in a position that the air flowing after gas element detection to pass the particulate matter measuring sensor unit and the particulate matter sensor unit 20 is arranged in the region between the gas sensor unit 10 and an outlet 32 thereby, the concentration of a particulate matter of the air can be measured.

Herein, the gas sensor unit 10 can be arranged to be attached to a bottom surface or side of the housing unit 30 according to an embodiment of the present description. However, this is merely an embodiment hence, the gas sensor unit 10 can be arranged in any position where the inflowing air can flow out to the outlet 32 of the housing unit 30.

Likewise, according to an embodiment of the present description, the gas sensor unit 10 can detect specific gas elements included in the air and emit heat energy during this process. The air is moved by a factor including the heat energy and measures the concentration of particulate matters in the air with the particulate measuring sensor unit 20 included in the housing unit 30.

The particulate matter measuring sensor unit 20 may comprise a light source unit 21, light detecting unit 22 and calculating unit. First, the light source unit arranged in the housing unit 30 irradiates the light on the air moved by the gas sensor unit 10 and the light detecting unit 22 detects scattering light by particulate matters included in the air and generates corresponding electric signal and the light is irradiated from the light source unit 21. The additionally formed calculating unit (not shown) may calculate the concentration of particulate matters using electric signal that is measured through the light detecting unit 22.

The light source unit 21 according to an embodiment of the present description can irradiate light of predetermined strength on the air. Herein, the light is scattered by particulate matters when particulate matters are included in the air thereby, light irradiated from the light source unit 21 is distorted with a specific angle.

The light detecting unit 22 is configured to detect the scattered light. Generally, the light detecting unit 22 is arranged in a position where the light irradiated from the light source unit 21 is not directly detected. More preferably, the light detecting unit 22 can be arranged in a position that can detect light when scattering light is generated by particulate matters in the air. Through this, the particulate matter measuring sensor unit 20 can measure the concentration of the particulate matter with higher accuracy.

The light source unit 21 can apply all kinds of light source module that irradiates light in a predetermined direction. For example, any one selected from white light diode, infrared light LED diode, and laser diode can be a light source module. That is, the light source unit 21 can be selectively applied according to measuring method and standard of a particulate matter.

The light detecting unit 22 is an element that can detect a scattering light generated by particulate matters and can include a photo diode and etc.

Preferably, an embodiment of the present description may comprise the light source unit 21 and the light detecting unit 22, and further comprise optical lens and etc. thereby irradiated light can be converged or condensed and can increase efficiency of detecting the scattered light. According to an embodiment of the present description, a mirror configured to reflect the scattered light to the light detecting unit 22 direction.

FIG. 3 is merely an embodiment of the present description and the light source unit 21 and the light detecting unit 22 can be arranged in a position and structure that can easily measure a concentration of particulate matter in the air unlike FIG. 3.

For example, referring to FIG. 3, when the gas sensor unit 10 is arranged in the bottom surface of the housing unit 30, the light source unit 21 and the light detecting unit 22 can be respectively arranged in the left or right side surface of the housing unit 30. Herein, the light source unit 21 and the light detecting unit 22 can be arranged tilted in a predetermined angle to measure scattering light due to particulate matters.

The afore-mentioned particulate matter measuring sensor unit 20 measures particulate matter concentration through the module and the air passed through the particulate matter measuring sensor unit 20 is emitted outside through the outlet 32.

Herein, a pump (not shown) configured to generate air flow for outside air to flow in to an inlet 31 of the housing unit 30, can be additionally formed. The pump is configured for the outside air to easily flow into the housing unit 30 thereby, a hybrid environment sensor according to the present description can easily measure gas elements included in the air and particulate matter concentration.

The hybrid environment sensor likewise can be combined with a portable device and FIG. 4 illustrates thereof.

FIG. 4 illustrates a diagram of a hybrid environment sensor according to an embodiment of the present description.
As illustrated in FIG. 4, the hybrid environment sensor according to an embodiment of the present description can be combined with a portable device 40 comprising the light source unit 41 and a camera module 42.

First, the portable device 40 may comprise a light source unit 41 and a camera module 42, hence can be any device capable of storage and analysis of image information such as smartphones like I-phone, android phone and etc.

Herein, FIG. 4 shows that the light source unit 41 and the camera module 42 is horizontally separated in a predetermined space thereby, the light induction unit 33 and light detecting induction unit 34 can be horizontally separated in a predetermined space. However, the hybrid environment sensor according to the present description can adaptively change according to arrangement structure of the light source unit and the camera module.

Herein, the portable device 40 can obtain comprehensive environment information regarding the surrounding air through obtaining the concentration of particulate matters detected through light induction unit 33 of the housing 30 and light detecting induction unit 34.

Herein, the housing unit 30 is combined with the portable device and includes the inlet 31 configured for the air to flow in and the outlet 32 configured for the inflow air to emit.

Herein, the gas sensor unit 10 is arranged in the housing unit 30 and the gas sensor unit 10 generates heat energy during operation process thereby, the air flowing in through the inlet 31 flows and emits to the outlet 32 of the housing unit 30.

According to an embodiment of the present description, a hybrid environment sensor according to the present description does not include a specific light source unit and a light detecting unit but measures the concentration of particulate matters in the air by using the light source unit 41 and the camera module 42 included in the portable device 40.

For this, the light induction 33 and the light detecting induction unit 34 can be additionally formed in the housing unit 30.

First, the light induction unit 33 is configured to induce the light irradiated from the light source unit 41 of the portable device 40 in the housing unit 30. For this, the light induction unit 33 can closely combine with the light source unit 41 not to leak the light of the light source unit 41 and the light induction unit 33 is comprised of optical fiber thereby, the light of the light source unit 41 can be induced in the housing unit 30.

The light induction 33 according to an embodiment of the present description may be formed with a plurality of mirrors modules. Further, any technical feature that can induce the light irradiated from the light source unit 41 in the housing unit 30.

The light induction unit 33 can induce the light irradiated from the light source unit 41 of the portable device 40 thereby, can be irradiated on the light that is flowing by the heat generated from the gas sensor unit 10.

Herein, the light induction unit 33 may additionally include a lens module. As illustrated in FIG. 4, the light induced from the light source unit 41 can be concentrated and irradiated on a predetermined region.

The light detecting induction unit 34 is combined with a camera module 42 of the portable device 40 thereby, the light scattered by particulate matters of the light irradiated by the light induction unit 33 can be induced to be detected by a camera module 42 of the portable device 40.

More preferably, the light detecting induction unit can improve detecting accuracy of the scattering light by including a lens module that collects the light that is scattered by the particulate matters included in the air.

The portable device 40 can measure the concentration of particulate matters in inflow air using scattering light information detected by the camera module 42 through the light detecting induction unit 34. For this, the portable device 40 can use programs of self-algorithm and etc. and the calculation can be implemented in an execution processing unit such as CPU (Central Processing Unit) and etc.

That is, the portable device 40 can measure the concentration of particulate matters based on electric signals generated through the camera module 42 detecting light through the light detecting induction unit 34.

Further, the gas sensor unit 10 can provide sensing information with the portable device 40 with an additional communication module. For example, the gas sensor unit 10 can provide sensing information with a wired communication through an earphone connection terminal formed on the portable device 40 or through an additional wireless communication module such as Bluetooth, NRC and etc. formed in the gas sensor unit 10.

A hybrid environment sensor illustrated in FIG. 3 and FIG. 4, can detect concentration of particulate matter in the surrounding air with gas elements using technical features minimized compared to a related art. The information of measured gas element and particulate concentration can be detected and managed comprehensively through wired or wireless communication of a portable device or an additional processing device and such information can be provided to a user through a display unit of the portable device or an additional display device.

As discussed, embodiments of the invention can provide a hybrid environment sensor comprising: a gas sensor unit configured to detect at least one gas element included in an inflowing air; and a particulate matter measuring sensor unit configured to measure a concentration of the particulate matter in the air moved by the hybrid environment sensor.

In some embodiments, heat energy produced by the gas sensor can move the air. In other embodiments, the air can be moved by an additionally equipped pump, or by a combination of additionally equipped pump and heat energy produced by the gas sensor.

While this disclosure includes specific examples, it will be apparent to one of ordinary skill in the art that various changes in form and details may be made in these examples without departing from the scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner, and/or replaced or supplemented by other components or their equivalents. Therefore, the scope of the disclosure is defined not by the detailed description, but by the claims and their equivalents, and all variations within the scope of the claims and their equivalents are to be construed as being included in the disclosure.

## Claims

1. A hybrid environment sensor comprising:
a gas sensor unit configured to detect at least one gas element included in an inflowing air; and
a particulate matter measuring sensor unit configured to measure a concentration of the particulate matter in the air moved by the gas sensor unit.

2. The hybrid environment sensor of claim 1, wherein the gas sensor unit comprises a gas sensor module configured to detect at least one gas element that is selected from a group including a Volatile Organic Compound, VOC, Nitrogen Oxide, NOₓ, carbon monoxide, CO, carbon dioxide, CO₂, sulfur dioxide SOₓ, ozone, O₃.

3. The hybrid environment sensor of claim 1 or 2, wherein the gas sensor unit comprises a micro-heater.

4. The hybrid environment sensor of any one of claims 1 to 3, further comprising:
a housing unit comprising an inlet and an outlet;
wherein the air is arranged to flow into the inlet and emits from the outlet, and
the gas sensor unit and particulate matter measurement sensor unit are arranged in the housing unit.

5. The hybrid environment sensor of claim 4, wherein:
the gas sensor unit is arranged to detect at least one gas element that is included in the air flowing through the inlet of the housing unit, and
the particulate matter measuring sensor unit is arranged in the region between the gas sensor unit and the outlet.

6. The hybrid environment sensor of claim 5, wherein the gas sensor unit is attached to a bottom surface or side surface of the housing unit.

7. The hybrid environment sensor of any one of claims 4 to 6, further comprising:
a pump configured for the air to be pumped into the inlet.

8. The hybrid environment sensor of any one of claims 1 to 7, wherein the particulate matter measurement sensor unit comprises:
a light source unit;
a light detecting unit configured to generate an electric signal corresponding to a detected light scattered by the particulate matter included in the air, and the light is emitted from the light source unit; and
a calculating unit configured to calculate a concentration of the particulate matter by using the electric signal that is detected through the light detecting unit.

9. The hybrid environment sensor of claim 8, wherein the light source unit comprises at least one that is selected from a white light diode, an infrared LED diode, and a laser diode.

10. The hybrid environment sensor of any one of claims 1 to 7, wherein the particulate matter measurement sensor unit comprises:
a filter configured to filter the particulate matter in the air; and
a calculating unit configured to calculate the concentration of the particulate matter using weight information of the particulate matter that is filtered by the filter.

11. The hybrid environment sensor of any one of claims 1 to 10, wherein:
the gas sensor unit is arranged to generate heat energy in an operation process and,
the air is arranged to be moved at least in part by the heat energy.

12. A hybrid environment sensor of claim 4 or any claim when dependent on claim 4, comprising:
a light induction unit configured to induce irradiation of the light which is irradiated from a light source unit of a portable device including a light source unit and a camera module to the air flowing by the gas sensor unit;
a light detecting induction unit configured to induce the camera module of the portable device to detect a light which is irradiated from the light induction unit thereby scatters by a fine-dust included in the air,
the housing unit configured to be combined with the portable device, and
the gas sensor unit configured to provide the gas element related information to the portable device.

13. The hybrid environment sensor of claim 12, wherein the light detecting induction unit comprises a lens module configured to converge light that is scattered by fine-dust included in the air.
